# EUROPÄISCHE PATENTANMELDUNG

(11) **EP 1 842 587 A1**
(43) Veröffentlichungstag der Anmeldung: **10.10.2007**
(21) Anmeldenummer: 06112172.9
(22) Anmeldetag: 03.04.2006
(51) Int. Cl.: B01J 19/00, G01N 31/10, G01N 21/31, G01N 25/18, G01N 25/72

(54) **Verwendung der Infrarot-Thermographie als Mittel zur Bestimmung des Aushärtungsverlaufes einer zweikomponentigen Zusammensetzung**

(71) Anmelder: Sika Technology AG, 6340 Baar (CH)
(72) Erfinder: Buck, Manuel, 5412, Gebenstorf (CH); Cirillo, Fabio, 8406, Winterthur (CH)

(57) **Zusammenfassung**

Die vorliegende Erfindung betrifft einerseits die Verwendung von Infrarot-Thermografie als Mittel zur Bestimmung des Aushärtungsverlaufes einer zweikomponentigen Zusammensetzung. Insbesondere lassen sich Mischfehler frühzeitig erkennen und als Mittel zur In-Prozess-Kontrolle für die Optimierung des Dosierverhältnisses der zwei Komponenten sowie für das Erkennen des Endes der Offenzeit einsetzen. Durch eine derartige Verwendung können mögliche Fehler frühzeitig, das heisst, vor dem Fügen oder Kontaktieren mit einer Substratoberfläche, erkannt werden, was zu einem schnelleren und sicheren Prozess sowie geringere Ausschuss- oder Aufarbeitungskosten führt.
Weitere Gegenstände der vorliegenden Erfindung sind somit auch eine Fertigungslinie, ein industriell gefertigter Artikel sowie ein Bauwerk oder Transportmittel.

## Beschreibung

### Technisches Gebiet

Die Erfindung betrifft das Gebiet der Aushärtung von zweikomponentigen Zusammensetzungen.

### Stand der Technik

Zweikomponentige Zusammensetzungen werden schon lange breit eingesetzt. Zweikomponentige Zusammensetzungen härten beim Mischen der zwei Komponenten aus. Aufgrund der schnellen Aushärtung einer Vielzahl von zweikomponentigen Zusammensetzungen finden sie in industriellen Prozessen breite Verwendungen. Für die Aushärtung von zweikomponentigen Zusammensetzungen, insbesondere von Klebstoffen, ist eine gute Mischung der zwei Komponenten sehr wichtig. Schlechtes Vermischen, sowie Dosierfehler, können starke Auswirkungen auf das Aushärten und somit auf die mechanischen Eigenschaften der ausgehärteten Zusammensetzung haben. Es ist somit für eine verlässliche Qualität sehr wichtig, die Güte der Vermischung sowie die Dosiergenauigkeit zu gewährleisten. Mischfehler sind auf verschiedene Ursachen zurückzuführen. Einerseits ist die Güte der Mischung von der Konsistenz der zwei Komponenten abhängig, andererseits ist sie sehr abhängig von den verwendeten Mischgeräten sowie der Mischzeit. Um beurteilen zu können, ob wirklich beide Komponenten vorhanden sind und wie gut sie miteinander gemischt werden, werden die zwei Komponenten vielfach mit zwei unterschiedlichen Farben eingefärbt. Die Farben werden hierbei üblicherweise derart gewählt, dass sich die Mischfarbe stark von den nicht gemischten Farben unterscheidet. So werden beispielsweise die Farbkombinationen weiss/schwarz, weiss/rot, weiss/blau oder blau/gelb für diesen Zweck verwendet. Das Auftreten von Farbunterschieden oder -Streifen lässt beispielsweise darauf schliessen, dass zuwenig lang gerührt wurde oder dass in einem Statikmischer zu wenig Mischelemente verwendet wurden. Dieses visuelle Beurteilungsverfahren ist aber einerseits sehr unempfindlich und andererseits lassen sich nur eine kleine Anzahl von Farbkombinationen unter Verwendung dieser Methode realisieren. Bei Abdichtungen oder sichtbaren Verklebungen, wie sie beim Verkleben von transparenten Materialien oder dekorativen Anwendungen vielfach auftreten, ist eine derartige Limitierung der Farbe der gemischten Zusammensetzung von grossem Nachteil. Insbesondere sind farblose oder helle Zusammensetzungen somit nicht oder nur sehr schlecht realisierbar.

Als Offenzeit einer zweikomponentigen Zusammensetzung wird die Zeit bezeichnet, die zwischen dem Mischen und dem Zeitpunkt verstreicht, bei welchem die Zusammensetzung aufgrund der fortgeschrittenen Vernetzung für den vorgesehenen Einsatz nicht mehr verwendbar ist. Bei einem Kleb- oder Dichtstoff, ist dieser Zeitpunkt derjenige, bei welchem die Zusammensetzung nicht mehr auf einer mit der Zusammensetzung kontaktierten Oberfläche haftet. Die Offenzeit ist somit ein limitierender Faktor in jedem Prozess, in welchem eine zweikomponentige Zusammensetzung zum Einsatz kommt. Leider ist ein Überschreiten der Offenzeit vielfach nicht sichtbar und wird unter Umständen erst bei einem Schadenfall offenkundig. Um derartige Schadensfälle zu verhindern, werden insbesondere Verklebungen und Dichtungen nach dem Aushärten mit grossem Aufwand geprüft. Als Methoden für eine derartige Qualitätsprüfung von Verklebungen werden insbesondere Ultraschall, Röntgenstrahlen sowie in neuerer Zeit auch Infrarot-Thermografie eingesetzt. Der Nachteil dieser Methoden ist jedoch, dass die Prüfung erst nach dem Aushärten erfolgt und nicht vor dem Fügen. Wenn sich somit beim Prüfen eines derartigen Verbundkörpers herausstellt, dass die Verklebung oder Dichtung Mängel aufweist, muss der ganze Verbundkörper entsorgt werden oder allenfalls mit grossem Aufwand getrennt und wieder in den Prozess eingegliedert werden. Dies führt vor allem bei teuren oder mechanisch delikaten Substraten zu grossen Verlusten.

### Darstellung der Erfindung

Die Aufgabe der vorliegenden Erfindung ist es daher, ein Verfahren zur Verfügung zu stellen, welches die Nachteile des Standes der Technik überwindet und insbesondere befähigt, den Aushärtungsverlauf einer zweikomponentigen Zusammensetzung zu bestimmen und insbesondere verlässliche Aussagen über die Mischgüte und die Offenzeit vor dem Fügen oder Kontaktieren mit einer Substratoberfläche zu erlauben.

Überraschenderweise wurde gefunden, dass gemäss Anspruch 1 die Infrarot-Thermografie ein derartiges Mittel darstellt, welches diese Aufgabe zu lösen im Stande ist.

Diese Methode ist insbesondere für industrielle Fertigung von Artikel, insbesondere für industriell verklebte Artikel, geeignet. Es ermöglicht die Güte einer Vermischung von zwei Komponenten frühzeitig zu beurteilen und allfällige Fehlstellen frühzeitig zu identifizieren.

Somit lässt sich auf einfache Weise der Aushärtungsverlauf bestimmen. Insbesondere lassen sich Mischfehler frühzeitig erkennen und als Mittel zur In-Prozess-Kontrolle für die Optimierung des Dosierverhältnisses der zwei Komponenten sowie das Erkennen des Endes der Offenzeit einsetzen. Durch eine derartige Verwendung können mögliche Fehler frühzeitig, das heisst, vor dem Fügen oder Kontaktieren mit einer Substratoberfläche, erkannt werden, was zu einem schnelleren und sicheren Prozess sowie geringere Ausschuss- oder Aufarbeitungskosten führt.

Weitere Gegenstände der vorliegenden Erfindung sind somit auch eine Fertigungslinie gemäss Anspruch 12, ein industriell gefertigter Artikel gemäss Anspruch 17 sowie ein Bauwerk oder Transportmittel gemäss Anspruch 19.

Bevorzugte Ausführungen der Erfindung sind Gegenstand der abhängigen Ansprüche.

### Wege zur Ausführung der Erfindung

Die vorliegende Erfindung betrifft einerseits die Verwendung von Infrarot-Thermografie als Mittel zur Bestimmung des Aushärtungsverlaufes einer zweikomponentigen Zusammensetzung.

Die hierfür verwendete zweikomponentige Zusammensetzung besteht aus zwei Komponenten K1 und K2. Grundsätzlich eignen sich alle zweikomponentigen Zusammensetzungen.

In einer bevorzugten ersten Variante weist hierbei die erste Komponente K1 mindestens eine Verbindung mit mindestens zwei funktionellen Gruppen X auf und die zweite Komponente K2 weist mindestens eine Verbindung mit mindestens zwei funktionellen Gruppen Z auf, wobei die funktionelle Gruppe X und die funktionelle Gruppe Z chemisch, insbesondere über eine Additionsreaktion, miteinander reagieren.

Die funktionelle Gruppe X ist insbesondere ausgewählt aus der Gruppe umfassend NCO, Epoxy, (Meth)acrylsäure, (Meth)acrylat und Alkoxysilan und die Gruppe Z ist insbesondere ausgewählt aus der Gruppe umfassend NH₂, NH, SH und OH.

In einer bevorzugten Ausführungsform enthält die erste Komponente K1 mindestens ein Polyisocyanat oder mindestens ein Isocyanatgruppen-terminiertes Polyurethanprepolymer und die zweite Komponenten K2 enthält mindestens ein Polyol oder ein Polyamin. Derartige Zusammensetzungen sind dem Fachmann auch als zweikomponentige Polyurethanzusammensetzungen bekannt.

Die Vorsilbe "Poly" in Substanzbezeichnungen, wie "Polyol", "Polyamin", "Polyglycidylether" "Polymercaptan" oder "Polyisocyanat" weist im vorliegenden Dokument darauf hin, dass die jeweilige Substanz formal mehr als eine der in ihrer Bezeichnung vorkommenden funktionellen Gruppe pro Molekül enthält.

In einer weiteren bevorzugten Ausführungsform enthält die erste Komponente K1 mindestens ein Polyisocyanat oder mindestens ein Isocyanatgruppen-terminiertes Polyurethanprepolymer und die zweite Komponenten K2 enthält mindestens Wasser.

In einer weiteren bevorzugten Ausführungsform enthält die erste Komponente K1 mindestens einen Polyglycidylether und die zweite Komponenten K2 enthält mindestens ein Polyamin oder ein Polymercaptan. enthält Als hierfür bevorzugten Polyglycidylether gelten Diglycidylether von Bisphenol-A und Bisphenol-F sowie deren Mischungen. Derartige Zusammensetzungen sind dem Fachmann auch als zweikomponentige Epoxidharzzusammensetzungen bekannt.

In einer bevorzugten zweiten Variante enthält die erste Komponente K1 mindestens eine Verbindung, welche unter dem Einfluss eines Katalysators oder Initiators, welcher in der zweiten Komponente K2 vorhanden ist, polymerisiert.

In eine bevorzugten Ausführungsform dieser Variante ist die unter dem Einfluss eines Katalysators oder Initiators der Komponente K2 polymerisiernde Verbindung eine ungesättigte Verbindung, welche ausgewählt ist aus der Gruppe bestehend aus Styrol, Acrylonitril, (Meth)acrylamide, (Meth)acrylsäure, (Meth)acrylate, Vinylalkohole, Vinylether, Vinylester, und ungesättigte Polyester und ist bevorzugt ein (Meth)acrylat. Die zweite Komponente K2 dieser Ausführungsform enthält als Initiator ein Radikalbildner, insbesondere ein Peroxid oder Hydroperoxid oder ein Perester, bevorzugt ein organisches Peroxid.

In gewissen Fällen, zum Beispiel, wenn die zweite Komponente einen Katalysator oder Initiator enthält, kann durch die Variation des Verhältnisses von K1 zu K2 im gewissen Rahmen die Offenzeit eingestellt, d.h. für den Klebeprozess optimiert, werden. Der Rahmen, in welchem die Topfzeit variiert werden kann, ohne dass die mechanischen Eigenschaften der ausgehärteten Zusammensetzung ungebührlich verschlechtert werden, ist sehr stark abhängig von der jeweiligen zweikomponentigen Zusammensetzung.

Die Komponenten K1 zu K2 können noch weitere Bestandteile enthalten, wie sie dem Fachmann für zweikomponentige Zusammensetzungen bekannt sind. Derartige weitere Bestandteile sind insbesondere Zusätze wie Weichmacher, Füllstoffe, Haftvermittler, UV-Absorptionsmittel, UV- oder und Wärmestabilisatoren, Antioxidantien, Flammschutzmittel, optische Aufheller, Katalysatoren, Farbpigmente oder Farbstoffe. Insbesondere bevorzugt als weitere derartige Bestandteile sind Füllstoffe. Als bevorzugte Füllstoffe gelten Russ (Carbon black) und Kreiden, sowohl beschichtet als auch unbeschichtet.

Als Thixotropiermittel gelten insbesondere pyrogene Kieselsäuren oder Harnstoffderivate, wie sie beispielsweise in EP-A-1 152 019 offenbart werden.

Es wird bevorzugt, dass die Zusammensetzung, beziehungsweise die Komponenten K1 und K2, eine pastöse Konsistenz aufweisen. Dies wird vor allem durch die Verwendung von Füllstoffen und/oder Thixotropiermittel als zusätzliche Bestandteile erreicht. Denn es treten nämlich vor allem bei pastösen Zusammensetzungen Probleme mit der Mischgüte auf, so dass vor allem für diese hier beschriebene Verwendung der Thermografie als Mittel zur Bestimmung des Aushärtungsverhaltens sehr vorteilhaft ist.

Die Infrarot-Thermografie wird als Mittel zur Bestimmung des Aushärtungsverlaufes einer vorgängig beschriebenen zweikomponentigen Zusammensetzung verwendet.

Mit dieser Methode können einerseits die Offenzeit und andererseits die Mischgüte bestimmt werden.

Da die Aushärtung einer zweikomponentigen Zusammensetzung ein exothermer Prozess ist, kann die bei der Aushärtung entstehende Wärme mittels IR-Thermographie, insbesondere über eine Infrarot-Kamera, detektiert und ausgewertet werden. Da diese IR-Thermografie vorzugsweise ein Wärmebild einer Oberfläche darstellt, kann aus der räumlichen und zeitlichen Wärmedetektierung der Aushärtungsverlauf bestimmt werden.

Bei Vorliegen einer homogenen Wärmeverteilung über die Oberfläche einer gemischten zweikomponentigen Zusammensetzung liegt eine gute Mischungsgüte vor, während das Auftreten von "Hot Spots" eine schlechte Vermischung als Ursache hat. Somit kann aus dem Verfolgen und Vergleichen der örtlichen und zeitlichen Veränderungen der Wärme die Mischgüte bestimmt werden.

Da durch die Vernetzungsreaktion Wärme entsteht, kann diese zum bestimmen des Endes der Offenzeit über die Temperaturentwicklung bestimmt werden. Am Anfang besitzt die Zusammensetzung die Anfangstemperatur T₀. Nach dem Mischen der zwei Komponenten ist während einer Latenzzeit tₓ im Wesentlichen kein Temperaturanstieg feststellbar. Zu Beginn der Vernetzung entsteht eine kleine Wärmetönung der Zusammensetzung, welche bei fortschreitender Vernetzung bis zu einer Maximaltemperatur Tₘₐₓ immer stärker wird. Nach dem Ende der Reaktion kühlt sich die Zusammensetzung langsam wieder ab. Durch die Wärmedissipation bedingt, ist jedoch beim Erreichen des Wärmemaximums Tₘₐₓ die Offenzeit bereits überschritten. Welcher Messtemperatur das Ende der Offenzeit (t_{oz}) beschreibt, ist jedoch stark abhängig von der individuellen zweikomponentigen Zusammensetzung. Diese Temperatur hängt unter anderem von der chemischen Reaktivität, der Umgebungstemperatur sowie der Chemie der zweikomponentigen Zusammensetzung ab. Diese Temperatur kann jedoch durch eine Korrelation mit einer physikalischen Prüfmethode, zum Bespiel der Konsistenz, der Klebrigkeit und/oder der Viskosität bestimmt werden.

Somit kann durch die Verfolgung der Temperaturen über die gesamte beobachtete Oberfläche für eine Zusammensetzung beim Feststellen einer Abkühlung nach dem Erreichen der Maximaltemperatur ein Überschreiten der Offenzeit festgestellt werden.

Zur Infrarot-Thermografie wird vorzugsweise eine Infrarot-Kamera eingesetzt. Bevorzugt ist diese IR-Kamera mit einem Computer verbunden. Auf dem Computer läuft bevorzugt ein Computer-Programm, welches die von der Kamera an den Computer übermittelte Information des Wärmebildes analysiert und nach vorgegebenen Schwellwerten einen Alarm oder ein Signal an eine Klebstoffdosiereinheit und/oder an eine Entnahmestation sendet.

Zur IR-Thermographie eigenen sich insbesondere Apparate, welche befähigt sind, Temperaturen im Bereich zwischen -20°C und 200°C, insbesondere zwischen 0°C und 150 °C, zu erfassen. Als sehr vorteilhaft gezeigt haben sich Apparaturen, welche befähigt sind, über einen längeren Zeitraum mit einer hohen Zeitauflösung zu arbeiten. Je nach Reaktivität der Zusammensetzung sind Beobachtungsräumen von bis zu mehreren Stunden, typischerweise von bis zu 20 Minuten, mit einer Frequenz von bis zu 30 Bilder pro Sekunden gefordert. Neben einer hohen Ortsauflösung ist weiterhin eine hohe Temperaturempfindlichkeit von grossem Vorteil. Als besonders geeignete IR-Kamera hat sich die Wärmebildkamera MIDAS 320 von DIAS Infrared GmbH, Deutschland, für diesen Zweck erwiesen.

Die Infrarot-Thermografie als Mittel für die Bestimmung des Aushärteverhaltens kann grundsätzlich für jede Applikation einer oben beschriebenen zweikomponentigen Zusammensetzung verwendet werden. Insbesondere eignet sie sich für Anwendungen der zweikomponentigen Zusammensetzung als Bodenbeläge, Anstriche, Beschichtungen, Dichtstoffe oder Klebstoffe. Als besonderes bevorzugt ist jedoch deren Anwendung als Klebstoffe. Insbesondere bevorzugt wird ein Verfahren zur Herstellung eines industriell verklebten Artikels, bei welchem für das Verkleben Infrarot-Thermografie gemäss einer Verwendung, wie sie oben beschrieben wurde, als Mittel zur Bestimmung des Aushärtungsverlaufes verwendet wird.

Aus der Bestimmung des Aushärteverlaufes kann die Prozesszeit einer Verklebung optimiert werden. Für schnelle Fertigungsprozesse werden schnelle Klebstoffe bevorzugt. Bei grossflächigen oder aufwändigen komplexen Verklebungen ist jedoch vielfach die Offenzeit des Klebstoffs ein kritischer Faktor. Durch die Kenntnis und Steuerung der Offenzeit kann die Prozesszeit derart optimiert werden, dass das Fügen innerhalb der Offenzeit erfolgt und somit ein verlässlicher Verbund gewährleistet wird. So kann einerseits die Offenzeit des Klebstoffes auf die vorgegebenen Taktzeiten eines Fertigungsprozesses angepasst werden oder aber die Taktzeit kann auf die Offenzeit eines Klebstoffes angepasst werden.
Das Verfahren des Verklebens umfasst die folgenden Schritte
- Auftragen eines Klebstoffs, dessen erste Komponente K1 und zweite Komponente K2 miteinander mittels Mischer gemischt worden sind, auf eine Oberfläche eines Substrates S1
- Überwachung des Aushärtungsverlaufes mittels Thermografie
- Kontaktieren des Klebstoffs mit der Oberfläche eines weiteren Substrates S2 vor dem Ende der Offenzeit

Nach dem Kontaktieren setzt sich die Vernetzung des Klebstoffes fort und es entsteht ein verklebter Artikel.

Die Art der Substrate S1 und S2 kann sehr unterschiedlich sein. Sie können gleich oder verschieden voneinander sein. Bevorzugte Substrate sind Kunststoffe, insbesondere thermoplastische Kunststoffe, Gläser, Keramiken, Metalle und deren Legierungen, auf natürlichen Materialien basierende Werkstoffe wie Holz, Spanplatten, sowie Lacke. Als meist bevorzugte Substrate gelten lackierte Untergründe, wie lackierte Metallflansche, Kunststoffe, insbesondere PVC, sowie Glas, insbesondere mit Keramik beschichtetes Glas.

Die Mischung der zwei Komponenten kann über Statik- oder dynamische Mischer erfolgen. Die Verwendung eines dynamischen Mischers hat den Vorteil, dass auf einfache Art und Weise die Mischintensität variiert werden kann, zum Beispiel, wie weiter unten beschrieben, über ein Signal von einer Thermografiestation verursacht. Der Klebstoffauftrag erfolgt üblicherweise als Klebstoffraupe, vorzugsweise als Dreieckraupe.

Die Förderung und die entsprechend korrigierte Dosierung der zwei Komponenten des Klebstoffs kann über eine 2-Komponenten-Kartuschenpistole, welche manuell, hydraulisch oder pneumatisch betrieben werden kann, über eine 2-Komponenten-Kolbenpumpe/-Dosierer, über eine 2- Komponenten-Zahnradpumpe/-Dosierer erfolgen.

Somit bildet auch ein industriell verklebter Artikel, welcher nach einem oben beschriebenen Verfahren hergestellt wurde, einen weiteren Aspekt der vorliegenden Erfindung.

Dieser Artikel ist insbesondere eine Tür oder ein Fenster oder ein Transportmittel oder ein Anbau- oder Einbaumodul eines Transportmittels. Bevorzugt stellt es ein Automobil dar, dessen Scheibe beispielsweise über das beschriebene Verfahren hergestellt wurde.

Weiter bevorzugt ist dieser Artikel ein Fenster oder eine Tür. Besonders bei grossflächige Fenster oder Türen weisen die applizierten Klebstoffraupen eine grosse Länge auf. Durch die Länge bedingt, verstreicht zwischen Anfangspunkt und Endpunkt der Klebstoffraupe eine erhebliche Zeit. Nach dem Applizieren des Klebstoffs muss jedoch noch genügend Zeit für das Fügen sein. Es ist somit vor allem in diesen grossflächigen Klebeanwendungen kritisch, dass die Offenzeit an gewissen Orten in der Klebstoffraupe überschritten wird. Ein Überschreiten der Offenzeit bewirkt, dass an diesen Stellen der Klebverbund nicht gewährleistet ist und somit an diesen Stellen Schwachstellen in der Kraftübertragung und/oder Abdichtung vorhanden sind.

Die beschriebenen industriell verklebten Artikel können gelagert und transportiert werden. Aufgrund des Trends von der Fertigung weg vom Band hin ins Zulieferwerk wird die Verwendung von An- und Einbaumodule, welche an einem anderen Ort als die Endfertigung hergestellt werden, zunehmend wichtiger.

In der Baubranche werden seit langem industriell gefertigte An- und Einbaumodule verwendet.

Somit bildet ein Bauwerk oder Transportmittel, welche einen wie oben beschriebenen industriell verklebten Artikel aufweisen, einen weiteren Aspekt der Erfindung.

Da die Verwendung der Infrarot-Thermografie als Mittel für die Bestimmung des Aushärteverhaltens vor allem für industrielle Fertigung von Artikeln bevorzugt ist, bildet eine Fertigungslinie für die Herstellung eines Artikels einen weiteren Aspekt der vorliegenden Erfindung.
Diese Fertigungslinie weist stromabwärts
a) mindestens eine Klebstoffapplikationsstation eines über einen Mischer gemischten zweikomponentigen Klebstoffes K bestehend aus einer ersten Komponenten K1 und einer zweiten Komponente K2,
b) mindestens eine Thermografiestation, und
c) mindestens eine Fügestation
auf.

"Stromabwärts" ist in diesem Zusammenhang als "zeitlich nacheinander abfolgend im Fertigungsprozess" zu verstehen. Um einen effizienten Ausstoss im Sinne einer industriellen Fertigungslinie zu gewährleisten, ist es klar, dass die Fertigungslinie nicht nur mit jeweils einem Artikel beschickt ist, sondern dass zu einem gegebenen Zeitpunkt jeweils unterschiedlich weit gefertigte Artikel jeweils an der jeweiligen Station vorhanden sind.

Die Fertigung ist vorzugsweise automatisch, was aber nicht ausschliesst, dass gewisse manuelle Stationen vorhanden sind, bzw. Handlungen durch Menschen ausgeführt werden können.

In einer Ausführungsform besteht zwischen der Thermografiestation und der Klebstoffapplikationsstation eine Kommunikationsverbindung. Insbesondere kann über diese Kommunikationsverbindung ein Signal übermittelt werden, aufgrund dessen das Verhältnis der Dosierung der Menge der ersten Komponente K1 zur zweiten Komponente K2 in der Klebstoffapplikationsstation verändert werden kann.

In einer weiteren Ausführungsform der Fertigungslinie ist zwischen Thermografiestation und Fügestation eine Entnahmestation angeordnet und es besteht eine Kommunikationsverbindung zwischen Thermografiestation und Entnahmestation. Insbesondere wird über diese Kommunikationsverbindung ein Signal übermittelt, aufgrund dessen ein Halbfabrikat, welches die Fertigungslinie bis zur Entnahmestation durchlaufen hat, der Fertigungslinie entnommen wird.

In einer weiteren Ausführungsform der Fertigungslinie ist zwischen Thermografiestation und Fügestation eine Entnahmestation angeordnet und es bestehen eine Kommunikationsverbindung zwischen Thermografiestation und Entnahmestation und zwischen der Thermografiestation und der Klebstoffapplikationsstation. Über diese Kommunikationsverbindungen können Signale übertragen werden aufgrund dessen das Verhältnis der Dosierung der Menge der ersten Komponente K1 zur zweiten Komponente K2 in der Klebstoffapplikationsstation verändert werden kann, bzw. aufgrund dessen ein Halbfabrikat, welches die Fertigungslinie bis zur Entnahmestation durchlaufen hat, der Fertigungslinie entnommen wird.

### Kurze Beschreibung der Zeichnung

Im Folgenden werden anhand der Zeichnungen ausgewählte Ausführungsbeispiele der Erfindung näher erläutert. Gleiche Elemente sind in den verschiedenen Figuren mit den gleichen Bezugszeichen versehen. Die Richtung von Kräften beziehungsweise Bewegungen ist mit Pfeilen angegeben.

Es zeigen:
- Fig. 1: eine schematische Aufzeichnung einer Fertigungslinie
- Fig. 2: eine schematische Aufzeichnung einer Fertigungslinie mit Optimierung des Dosierverhältnisses
- Fig. 3: eine schematische Aufzeichnung einer Fertigungslinie mit einer Entnahmestation
- Fig. 4: eine schematische Aufzeichnung einer Fertigungslinie mit Optimierung des Dosierverhältnisses und einer Entnahmestation
- Fig. 5: einen schematischen Querschnitt durch eine Fertigungslinie mit Optimierung des Dosierverhältnisses
- Fig. 6: einen schematischen Querschnitt durch eine Fertigungslinie mit Entnahmestation
- Fig. 7: Wärmebilder einer pneumatischen Applikation eines Methacrylatklebstoffes bei 10°C nach unterschiedlichen Zeiten nach Applikation
- Fig. 8: eine grafische Darstellung des Temperaturverlaufes an zwei in der Klebstoffraupe ausgewählten Punkten (Punkte 3 und 4) in Figur 7
- Fig. 9: Wärmebilder einer manuellen Applikation eines Methacrylatklebstoffes bei 10°C nach unterschiedlichen Zeiten nach Applikation
- Fig. 10: eine grafische Darstellung des Temperaturverlaufes an zwei in der Klebstoffraupe ausgewählten Punkten (Punkte 3 und 4) in Figur 9
- Fig. 11: einen dreidimensionale Darstellung des Temperaturverlaufes entlang der Linie 1 (pneumatische Applikation) (hinten) und entlang der Linie 2 (manuelle Applikation) (vorne) auf Klebstoffraupen nach unterschiedlichen Zeiten t_{obs} nach der Applikation.

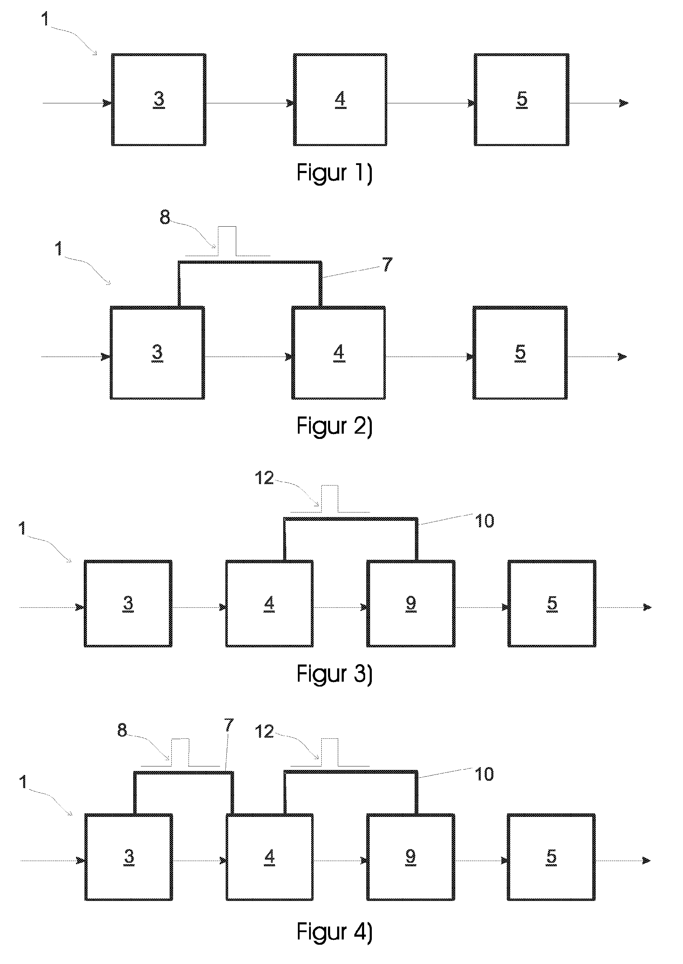

In Figur 1 ist schematisch eine Fertigungslinie 1 für die Herstellung eines Artikels 2 wiedergegeben. Diese Fertigungslinie weist stromabwärts aufeinander abfolgend die folgenden Stationen auf:
a) mindestens eine Klebstoffapplikationsstation 3
b) mindestens eine Thermografiestation 4, und
c) mindestens eine Fügestation 5

In der Klebstoffapplikationsstation 3 wird der über einen Mischer 6 gemischte zweikomponentige Klebstoff K, welcher aus einer ersten Komponenten K1 und einer zweiten Komponente K2 besteht, appliziert.

In Figur 2 ist eine bevorzugte Ausführungsform der in Figur 1 beschrieben Fertigungslinie 1 für die Herstellung eines Artikels 2, nämlich einer Fertigungslinie mit einer Optimierung des Dosierverhältnisses, beschrieben. Hierbei besteht zwischen Thermografiestation 4 und Klebstoffapplikationsstation 3 eine Kommunikationsverbindung 7. Über diese Kommunikationsverbindung 7 wird von der Thermografiestation ein Signal 8 an die Klebstoffapplikationsstation 3 übermittelt, aufgrund dessen das Verhältnis der Dosierung der Menge der ersten Komponente K1 zur zweiten Komponente K2 verändert werden kann.

Figur 3 beschreibt eine weitere bevorzugte Ausführungsform der in Figur 1 beschriebenen Fertigungslinie 1 für die Herstellung eines Artikels 2, nämlich einer Fertigungslinie mit einer Entnahmestation 9, welche zwischen Thermografiestation 4 und Fügestation 5 angeordnet ist. Hierbei besteht zwischen Thermografiestation 4 und Entnahmestation 9 eine Kommunikationsverbindung 10. Über diese Kommunikationsverbindung 10 wird von der Thermografiestation ein Signal 12 an die Entnahmestation 9 übermittelt, aufgrund dessen eine Entnahme eines Halbfabrikates 11, welches die Fertigungslinie bis zur Entnahmestation durchlaufen hat, ausgelöst wird.

In Figur 4 ist eine weitere bevorzugte Ausführungsform einer Fertigungslinie 1 dargestellt, welche im Wesentlichen eine Kombination der oben für Figur 2 und 3 beschriebenen Ausführungsformen darstellt, d.h., dass sowohl zwischen Thermografiestation 4 und Klebstoffapplikationsstation 3 eine Kommunikationsverbindung 7, als auch zwischen Thermografiestation 4 und Entnahmestation 9 eine Kommunikationsverbindung 10 bestehen. In dieser Ausführungsvariante kann sowohl das Verhältnis der Dosierung der Menge der ersten Komponente K1 zur zweiten Komponente K2 durch ein von der Thermografiestation 4 über die Kommunikationsverbindung 7 an die Klebstoffapplikationsstation 3 übermitteltes Signal 8 optimiert werden, als auch durch ein von der Thermografiestation 4 über die Kommunikationsverbindung 10 an die Entnahmestation 9 übermitteltes Signal 12 eine Entnahme eines Halbfabrikates 11 aus der Fertigungslinie 1 verursacht werden.

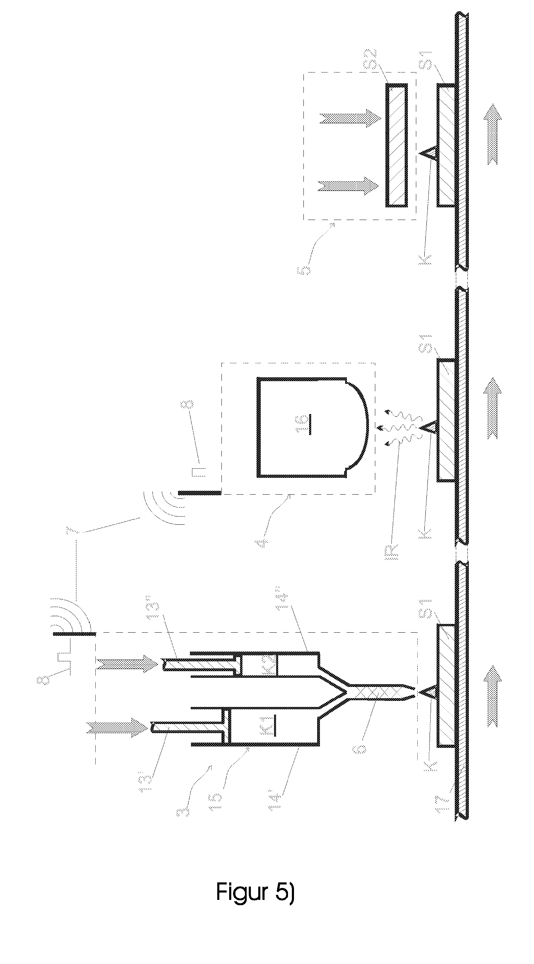

Figur 5 stellt einen schematischen Querschnitt durch eine Fertigungslinie zur Fertigung eines Artikels 2 mit Optimierung des Dosierverhältnisses dar. Die hier dargestellte Fertigungslinie 1 weist ein Förderband 17 auf, auf welchem ein Substrat S1 zuerst zu einer Klebstoffapplikationsstation 3, anschliessend zu einer Thermografiestation 4 und schliesslich zu einer Fügestation 5 bewegt wird. Bei der Klebstoffapplikationsstation 3 wird der über einen Mischer 6 aus den Komponenten K1 und K2 gemischte Klebstoff K auf die Oberfläche des Substrates S1, insbesondere als Dreiecksraupe, appliziert. In der hier dargestellten Ausführungsform erfolgt die Dosierung der ersten Komponente K1, beziehungsweise der zweiten Komponente K2, durch einen Dosierkolben 13' bzw. 13", aus einem Kartuschenzylinder 14', bzw. 14". Es ist dem Fachmann klar, dass anstelle der hier gezeigten Variante einer Dualkartusche 15, auch andere Förder- bzw. Dosiervorrichtungen verwendet werden können, wie beispielsweise Förderung/Dosierung mittels Kolbenpresse über Folgeplatte aus Hobbocks oder Fässern. Die Bewegung der Dosierkolben 13' und 13" erfolgt bevorzugt getrennt von einander, was die Optimierung der Dosierverhältnisse, bzw. Dosiermengen, erlaubt.

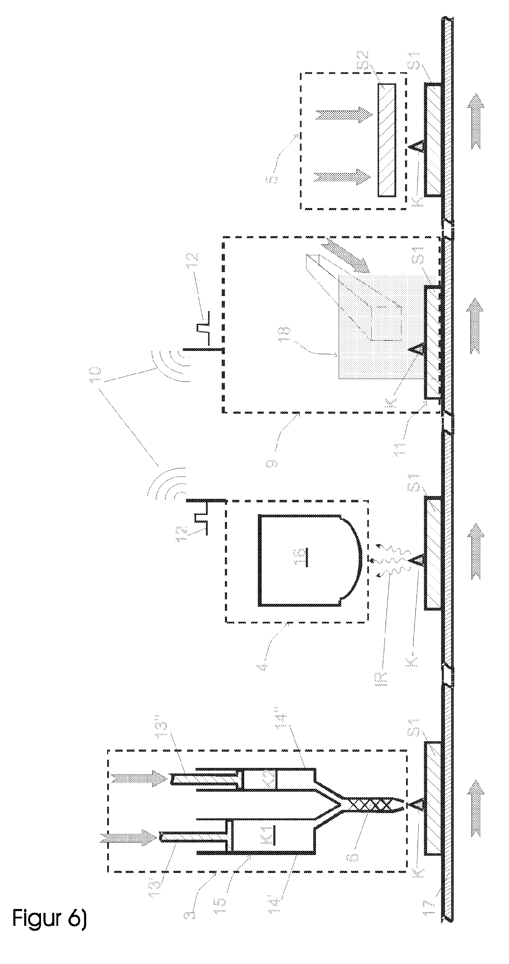

In einer weiteren Station im Herstellprozess eines Artikels wird die aufgrund der fortschreitenden Vernetzung des Klebstoffs K entwickelte Wärme, d.h. die IR-Strahlung in der Thermografiestation 4 erfasst und analysiert. In der hier gezeigten Darstellung ist in der Thermografiestation eine IR-Kamera 16 vorhanden. Die Analyse erfolgt in einem Computer (nicht dargestellt). Falls Mischfehler oder Dosierfehler entdeckt werden, löst der Computer ein Signal 8 aus, welches über eine Kommunikationsverbindung 7 an die Klebstoffapplikationsstation 3 übermittelt wird. In der hier dargestellten Form ist die Kommunikationsverbindung 7 eine Funkverbindung. Die Kommunikationsverbindung 7 kann jedoch auch ein Kabel sein. Die Kommunikationsverbindung 7 kann sowohl einseitig als auch zweiseitig sein. Durch das Signal 8 kann die Bewegungsart und/oder Bewegungsstrecke der Dosierkolben 13' resp. 13" gesteuert und dadurch optimiert werden. Es ist klar, dass beim Vorliegen eines starken Mischfehlers durch ein weiteres Signal bedingt eine manuelle oder automatische Entnahme, zum Beispiel wenn, wie in Figur 3 oder in Figur 6 dargestellt, eine Entnahmestation 9 Teil der Fertigungslinie ist, des Halbfabrikats ausgelöst werden kann.

In einer weiteren Station im Herstellprozess eines Artikels wird das Halbfabrikat, d.h. das Substrat S1 mit dem darauf applizierten Klebstoff K, mit einem weiteren Substrat, S2, in einer Fügestation 5 gefügt. Nach dem Fügen erfolgt die endgültige Vernetzung und es resultiert ein verklebter Artikel 2.

Figur 6 stellt einen schematischen Querschnitt durch eine Fertigungslinie zur Fertigung eines Artikels 2 mit einer Entnahmestation 9 dar.

Die hier dargestellte Fertigungslinie 1 weist ein Förderband 17 auf, auf welchem ein Substrat S1 zuerst zu einer Klebstoffapplikationsstation 3, anschliessend zu einer Thermografiestation 4, anschliessend zu einer Entnahmestation 9 und schliesslich zu einer Fügestation 5 bewegt wird. Bei der Klebstoffapplikationsstation 3 wird der über einen Mischer 6 aus den Komponenten K1 und K2 gemischte Klebstoff K auf die Oberfläche des Substrates S1, insbesondere als Dreiecksraupe, appliziert. In der hier dargestellten Ausführungsform erfolgt die Dosierung der ersten Komponente K1, beziehungsweise der zweiten Komponente K2, durch einen Dosierkolben 13' bzw. 13", aus einem Kartuschenzylinder 14', resp. 14". Es ist dem Fachmann klar, dass anstelle der hier gezeigten Variante einer Dualkartusche 15, auch andere Förder- bzw. Dosiervorrichtungen verwendet werden können, wie beispielsweise Förderung/Dosierung mittels Kolbenpresse über Folgeplatte aus Hobbocks oder Fässern. Die Bewegung der Dosierkolben 13' und 13" erfolgt bevorzugt getrennt von einander, was die Optimierung der Dosierverhältnisse, bzw. Dosiermengen, erlaubt.

In einer weiteren Station im Herstellprozess eines Artikels wird die aufgrund der fortschreitenden Vernetzung des Klebstoffs K entwickelte Wärme, d.h. die IR-Strahlung in der Thermografiestation 4 erfasst und analysiert. In der hier gezeigten Darstellung ist in der Thermografiestation eine IR-Kamera 16 vorhanden. Die Analyse erfolgt in einem Computer (nicht dargestellt). Falls das Überschreiten der Offenzeit festgestellt wird, löst der Computer ein Signal 12 aus, welches über eine Kommunikationsverbindung 10 an wird Entnahmestation 9 übermittelt. In der hier dargestellten Form ist die Kommunikationsverbindung 10 eine Funkverbindung. Die Kommunikationsverbindung 10 kann jedoch auch ein Kabel sein. Die Kommunikationsverbindung 10 kann sowohl einseitig als auch zweiseitig sein.

In einer weiteren Station im Herstellprozess eines Artikels kommt das Halbfabrikat 11 zur Entnahmestation 9. Diese Entnahmestation entnimmt gegebenenfalls beim Überschreiten der Offenzeit das Halbfabrikat aufgrund eines über die Kommunikationsverbindung 10 übermittelten Signals 12 das Halbfabrikat, welches die Fertigungslinie bis zur Entnahmestation durchlaufen hat, aus der Fertigungslinie. Zur Entnahme besitzt diese Entnahmestation 9 ein Entnahmemittel 18. Dieses Entnahmemittel 18 kann verschieden ausgebildet sein. Es kann sich beispielsweise um einen Robotergreifarm oder, wie in Figur 6 dargestellt, um einen Schieber 16 handeln, welcher das Halbfabrikat durch eine Bewegung quer zur Laufrichtung des Förderbandes 17 aus der Fertigungslinie 1 entfernt.

Falls die Offenzeit nicht überschritten ist, und damit das Entnahmemittel 18 nicht aktiviert wird, bleibt das Halbfabrikat in der Fertigungslinie und gelangt zu einer weiteren Station, der Fügestation 5. Dort wird das Halbfabrikat, d.h. das Substrat S1 mit dem darauf applizierten Klebstoff K, mit einem weiteren Substrat, S2, gefügt. Nach dem Fügen erfolgt die endgültige Vernetzung und es resultiert ein verklebter Artikel 2.

### Beispiele

Es wurde ein zweikomponentiger Klebstoff hergestellt, welche die in Tabelle 1 beschriebenen Inhaltsstoffe aufweist. Die Komponenten K1 und K2 wurden je in einen Kartuschenzylinder einer 200ml- Zwillingskartusche (10:1, Mixpac, Schweiz), respektive in zwei Hobbocks, abgefüllt.

**Tabelle 1. Klebstoff**

| | Gewichtsteile |
|---|---|
| Komponente K1 | |
| Tetrahydrofurfurylmethacrylat | 42 |
| Hycar VTBNX 1300 X33 | 21 |
| Paraloid EXL 2600 | 16 |
| p-Toluidine | 1 |
| Kreide | 20 |

| Komponente K2 | |
|---|---|
| Benzoylperoxidpaste (45% in Weichmacher) | 25 |
| Kreide | 25 |
| Extender | 35 |
| organisches Thixotropiermittel | 14 |
| schwarzes Pigment | 1 |

### Versuchsaufbau

Die folgenden Versuche erfolgten in einem auf die jeweilige Temperatur eingestellten Klimaschrank. Der über einen Statikmischer gemischte Klebstoff wurde als Klebstoffraupe eine Gummimatte als Isolationsschicht mit einer Raupenlänge von ca. 30 cm appliziert. Eine Infrarot-Kamera MIDAS 320 von DIAS Infrared GmbH, Deutschland, wurde auf einem Stativ in einem Abstand von circa 50 cm von den Klebstoffraupen fixiert, mit einem Laptop verbunden und mittels dem Computerprogramm Midas Spec R/T gesteuert. Der Aushärtungsverlauf wurde über die Aufnahme von Wärmebildern mit einem Bild pro Sekunde verfolgt. Dadurch, dass während der Aufnahme die Applikation im Bildablauf erkennbar ist, kann für einen jeweiligen beobachteten Punkt der Zeitpunkt t=0, d.h. der Applikationspunkt festgelegt werden.

### Aushärtungsverhalten

In einem ersten Experiment wurde die Mischgüte einer pneumatischen und einer manuellen Applikation des Klebstoffs in einem Mischungsverhältnis von K1 : K2 = 10 :1 bei 10°C verfolgt.

Das simultane Auspressen der beiden Zylinder der Kartusche geschieht entweder durch einen durch Muskelkraft betätigten Handabzug bei der manuellen Pistole oder durch einen pneumatisch (komprimierte Luft, 2.5 bar) betätigten Zylinder bei der pneumatischen Pistole. Bei der manuellen Version wird durch das Drücken und Loslassen des Handabzugs ein "Atmen" der Kartusche erzeugt.
Die zwei Klebstoffraupen wurden gleichzeitig nebeneinander aufgetragen. Die gesamte Raupe wurde innert 10 Sekunden aufgetragen.

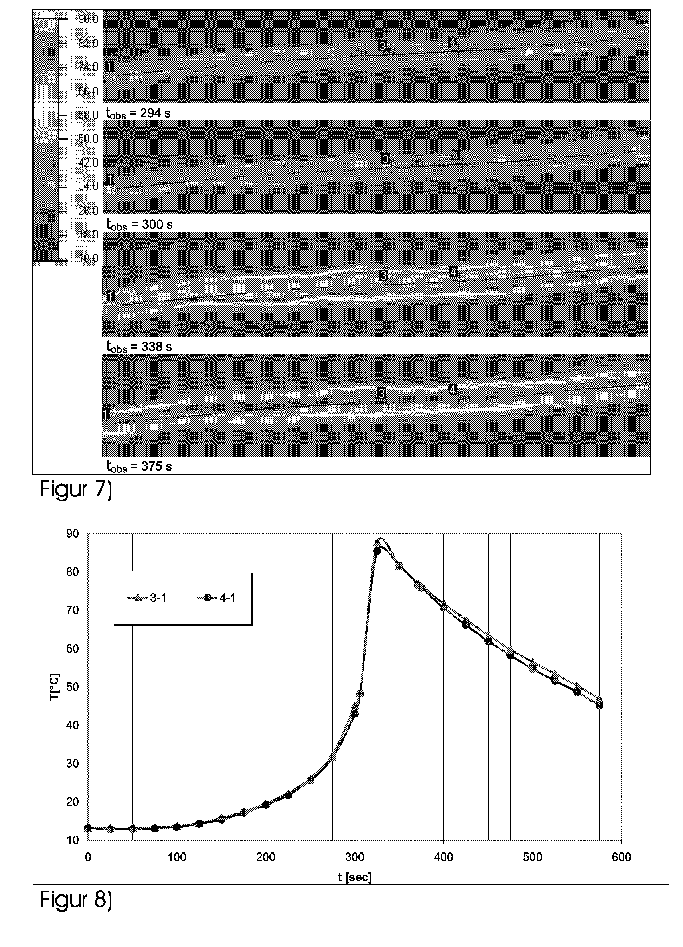

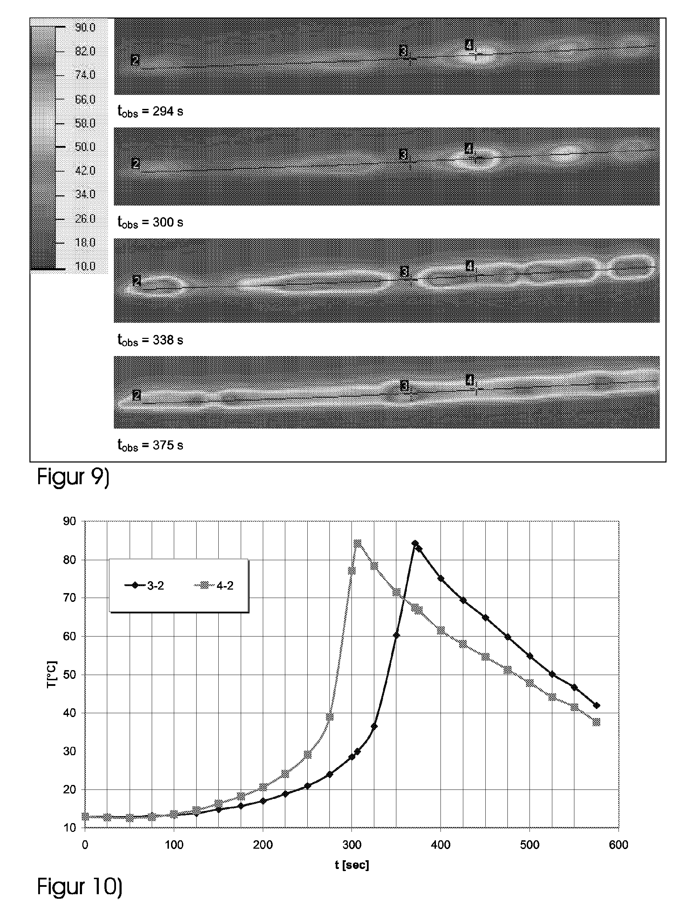

Figur 7, bzw. Figur 9 (Klebstoffauftrag von rechts nach links) zeigen die Wärmebilder für die Raupe mit der pneumatischen Applikation (Linie "1", in Figur 7), bzw. für die Raupe mit der manuellen Applikation (Linie "2", in Figur 9), bei einem Zeitpunkt ("t_{obs}") nach dem Klebstoffauftrag (gemessen bei Punkt 4) von 294 s, 300 s, 338 s und 375 s. Figuren 8 und 10 zeigen die zeitliche Entwicklung der Temperatur, welche an mit "3" und "4" bezeichneten Punkten in der jeweiligen Raupe (Linie "3" bzw. "4") gemessen wurde.

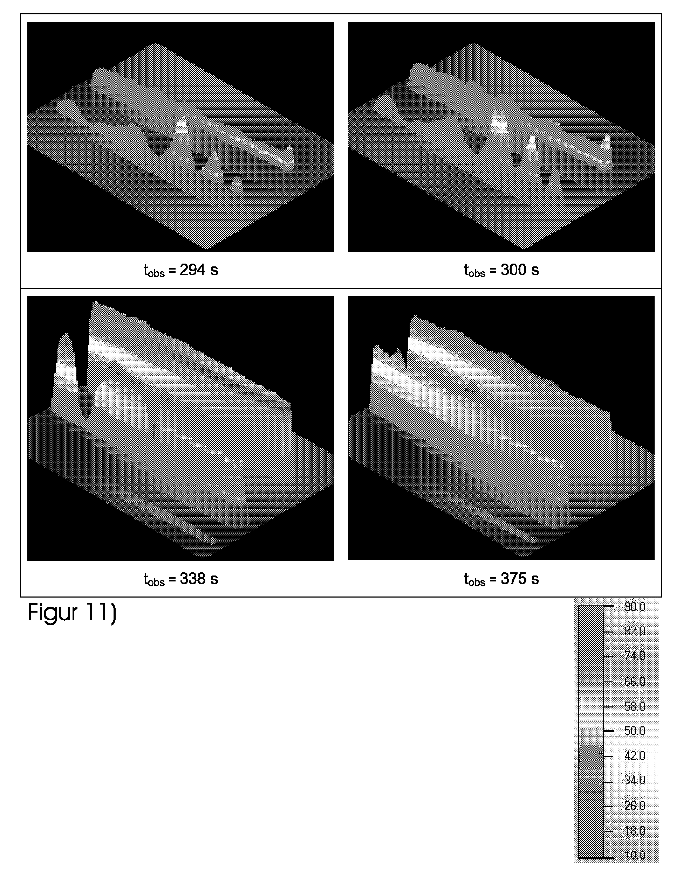

Figur 11 schliesslich zeigt eine dreidimensionale Darstellung der Wärmeverteilung entlang der Linien "1" und "2", wie sie in den Figuren 7 und 9 auf der Klebstoffraupe eingezeichnet sind.
In allen Wärmebildern wurde die gemessene Temperatur (°C) durch eine Farbe dargestellt entsprechend der angegebene Farbcodierung
Aus den Figuren 7 bis 11 geht hervor, dass im Vergleich der pneumatische zur manuellen Applikation das Aushärtungsverhalten der manuellen Applikation viel heterogener ist. Es treten innerhalb der Raupe starke Temperaturschwankungen auf. Für das Ende der Offenzeit wurde eine Temperatur von etwa 25°C bestimmt. Somit gibt es bei der manuellen Applikation Orte, bei denen diese Temperaturen bereits nach 250 Sekunden (Punkt 4) überschritten werden, während bei der pneumatischen Applikation diese Grenze erst bei 266 Sekunden (Punkt 3 und 4) überschritten wird. Der Zeitunterschied, bei der in den Punkten 3 und 4 der manuellen Applikation dieselbe Temperatur (T > 25°C) erreicht wird, beträgt ca. 55 Sekunden, während dies bei der pneumatischen Applikation lediglich 1 bis 2 Sekunden beträgt.

### Variation des Mischungsverhältnisses

Durch Variation des Verhältnisses von Komponente K1 zu Komponente K2 konnte die Offenzeit des Klebstoffes variiert werden. Das Dosierverhältnis wurde mit einer Anlage der Firma Failsafemetering, UK, auf die verschiedenen Dosierverhältnisse eingestellt. Das Pulsmeter System der Firma Failsafemetering erlaubt diese Einstellung durch Limitierung des Weges der Dosierkolben. Die eingestellten Materialdrücke von den Schöpfkolbenzuführpumpen aus jeweiligen Hobbocks waren für die erste Komponente K1 30 bar und für die zweite Komponente K2 50 bar .Bei diesem Experiment wurde der Klebstoff bei 20°C, wie oben beschrieben mit der pneumatischen Dosierung mit einem variablen Mischungsverhältnis appliziert. Als Ende der Offenzeit wurde die Temperatur von etwa 30°C ermittelt. Die Aushärtung wurde mittels Infrarot-Kamera verfolgt. Die Wärmebilder zeigten eine homogene Wärmeverteilung innerhalb der Raupen.

**Tabelle 2. Offenzeit als Funktion des Mischungsverhältnisses.**

| | | | | |
|---|---|---|---|---|
| Verhältnis K1/K2 | 6/1 | 8/1 | 10/1 | 13/1 |
| Offenzeit [s] | 76 | 77 | 82 | 90 |

### Bezugszeichenliste

- 1: Fertigungslinie
- 2: Artikel
- 3: Klebstoffapplikationsstation
- 4: Thermografiestation
- 5: Fügestation
- 6: Mischer
- 7,10: Kommunikationsverbindung
- 8,12: Signal
- 9: Entnahmestation
- 11: Halbfabrikat
- 13', 13': Dosierkolben
- 14', 14": Kartuschenzylinder
- 15: Zwillingskartusche
- 16: Infrarot-Kamera
- 17: Förderband
- 18: Entnahmemittel, Schieber
- IR: Infrarot-Strahlung
- K: Gemische zweikomponentige Zusammensetzung
- K1: erste Komponente
- K2: zweite Komponente
- S1: erstes Substrat
- S2: zweites Substrat

## Patentansprüche

1. Verwendung von Infrarot-Thermografie als Mittel zur Bestimmung des Aushärtungsverlaufes einer zweikomponentigen Zusammensetzung.

2. Verwendung gemäss Anspruch 1, **dadurch gekennzeichnet, dass** die zweikomponentigen Zusammensetzung aus einer ersten Komponente K1 und einer zweiten Komponente K2 besteht,
wobei die erste Komponente K1 mindestens eine Verbindung mit mindestens zwei funktionellen Gruppen X aufweist,
und die zweite Komponente K2 mindestens eine Verbindung mit mindestens zwei funktionellen Gruppen Z aufweist,
und wobei die funktionelle Gruppe X und die funktionelle Gruppe Z chemisch, insbesondere über eine Additionsreaktion, miteinander reagieren.

3. Verwendung gemäss Anspruch 2, **dadurch gekennzeichnet, dass** die erste Komponente K1 mindestens ein Polyisocyanat oder mindestens ein Isocyanatgruppen-terminiertes Polyurethanprepolymer enthält,
und die zweite Komponente K2 mindestens ein Polyol oder ein Polyamin enthält.

4. Verwendung gemäss Anspruch 2, **dadurch gekennzeichnet, dass** die erste Komponente K1 mindestens einen Polyglycidylether enthält,
und die zweite Komponente K2 mindestens ein Polyamin oder ein Polymercaptan enthält.

5. Verwendung gemäss Anspruch 1, **dadurch gekennzeichnet, dass** die zweikomponentige Zusammensetzung aus einer ersten Komponente K1 und einer zweiten Komponente K2 besteht,
wobei die erste Komponente K1 mindestens eine Verbindung enthält, welche unter dem Einfluss eines Katalysators oder Initiators, welcher in der zweiten Komponente K2 vorhanden ist, polymerisiert.

6. Verwendung gemäss Anspruch 5, **dadurch gekennzeichnet, dass** die erste Komponente K1 eine ungesättigte Verbindung, insbesondere eine ungesättigte Verbindung, welche ausgewählt ist aus der Gruppe bestehend aus Styrol, Acrylonitril, (Meth)acrylamide, (Meth)acrylsäure, (Meth)acrylate, Vinylalkohole, Vinylether, Vinylester, und ungesättigte Polyester, bevorzugt ein (Meth)acrylate, enthält;
und die zweite Komponente K2 ein Peroxid oder Hydroperoxid oder ein Perester, bevorzugt ein organisches Peroxid, enthält.

7. Verwendung gemäss einem der vorhergehenden Ansprüche, **dadurch gekennzeichnet, dass** für die Infrarot-Thermografie eine Infrarot-Kamera als Mittel zur Detektierung der Infrarot-Strahlung verwendet wird.

8. Verwendung gemäss einem der vorhergehenden Ansprüche, **dadurch gekennzeichnet, dass** aus der Bestimmung des Aushärtungsverlaufes die Offenzeit der zweikomponentigen Zusammensetzung bestimmt wird.

9. Verwendung gemäss einem der Ansprüche 2 bis 7, **dadurch gekennzeichnet, dass** aus der Bestimmung des Aushärtungsverlaufes das Dosierverhältnis der ersten Komponenten K1 und der zweiten Komponenten K2 optimiert wird.

10. Verfahren zur Herstellung eines industriell verklebten Artikels, bei welchem für das Verkleben Infrarot-Thermografie gemäss einer Verwendung nach einem der Ansprüche 1 bis 9 als Mittel zur Bestimmung des Aushärtungsverlaufes verwendet wird.

11. Verfahren gemäss Anspruch 10, **dadurch gekennzeichnet, dass** aus der Bestimmung des Aushärtungsverlaufes die Prozesszeit einer Verklebung optimiert wird.

12. Fertigungslinie (1) für die Herstellung eines Artikels (2), welche stromabwärts aufeinander abfolgend
a) mindestens eine Klebstoffapplikationsstation (3) eines über einen Mischer (6) gemischten zweikomponentigen Klebstoffes K bestehend aus einer ersten Komponenten K1 und einer zweiten Komponente K2,
b) mindestens eine Thermografiestation (4), und
c) mindestens eine Fügestation (5)
aufweist.

13. Fertigungslinie gemäss Anspruch 12, **dadurch gekennzeichnet, dass** zwischen Thermografiestation (4) und Klebstoffapplikationsstation (3) eine Kommunikationsverbindung (7) besteht.

14. Fertigungslinie gemäss Anspruch 13, **dadurch gekennzeichnet, dass** aufgrund eines Signals (8), welches über die Kommunikationsverbindung (7) übermittelt wird, das Verhältnis der Dosierung der Menge der ersten Komponente K1 zur zweiten Komponente K2 verändert wird.

15. Fertigungslinie gemäss einem der Ansprüche 12 bis 14, **dadurch gekennzeichnet, dass** zwischen Thermografiestation (4) und Fügestation (5) eine Entnahmestation (9) angeordnet ist und zwischen Thermografiestation (4) und Entnahmestation (9) eine Kommunikationsverbindung (10) besteht.

16. Fertigungslinie gemäss Anspruch 15, **dadurch gekennzeichnet, dass** die Entnahmestation (9) ein Halbfabrikat (11), welches die Fertigungslinie bis zur Entnahmestation durchlaufen hat, der Fertigungslinie aufgrund eines über die Kommunikationsverbindung (10) übermittelten Signals (12) entnimmt.

17. Industriell verklebter Artikel, welcher nach einem Verfahren gemäss Anspruch 10 oder 11 hergestellt wird.

18. Industriell verklebter Artikel gemäss Anspruch 17, **dadurch gekennzeichnet, dass** der Artikel eine Tür oder ein Fenster oder ein Transportmittel oder ein An- oder Einbaumodul eines Transportmittels ist.

19. Bauwerk oder Transportmittel aufweisend einen industriell verklebten Artikel gemäss Anspruch 17 oder 18.
